# FASCICULE DE BREVET EUROPEEN

(11) **EP 1 652 447 B1**
(45) Date de publication et mention de la délivrance du brevet: **07.05.2008**
(21) Numéro de dépôt: 05356190.8
(22) Date de dépôt: 25.10.2005
(51) Int. Cl.: A45D 26/00, A45D 29/05, A61B 17/54

(54) **Appareil à épiler comportant un dispositif de traitement de la peau**
Epilationsgerät mit einer Vorrichtung zur Hautbehandlung
Hair removal device comprising a skin treatment accessory

(30) Priorité: 28.10.2004 FR 0411524
(43) Date de publication de la demande: 03.05.2006
(73) Titulaire: SEB SA, 69130 Ecully (FR)
(72) Inventeur: Fabron, Jérome, 69970 Marennes (FR); Maisonneuve, Martial, 38090 Villefontaine (FR)
(74) Mandataire: Kiehl, Hubert

(56) Documents cités:
- WO-A-94/04116
- FR-A- 2 728 777
- FR-A- 2 833 470
- US-A1- 2002 107 527

## Description

La présente invention est relative à un appareil à épiler du type comportant un cylindre rotatif à pinces destinées à arracher les poils superflus du corps humain, et elle concerne plus particulièrement un dispositif de traitement de la peau pour un tel appareil.

Un appareil à épiler à pinces d'arrachage comporte en général des pinces sous forme de lames ou de disques disposés sur un cylindre rotatif, les pinces étant amenées à se fermer et s'ouvrir périodiquement de manière à saisir et serrer les poils à proximité de la peau pour les arracher de par la rotation du cylindre et ensuite à évacuer les poils arrachés avant de recommencer un nouveau cycle d'arrachage.

L'arrachage des poils s'avère une méthode très efficace contre leur repousse, mais, souvent, l'utilisatrice a besoin de préparer la peau avant de procéder à l'épilation. Notamment, les poils qui sont affaiblis par les arrachages successifs ont tendance à repousser sous la peau. Par conséquent, la couche cornée superficielle doit être éliminée au, préalable afin de libérer les poils incarnés. Ainsi, il s'est avéré qu'une opération de gommage de la peau effectuée avant l'arrachage permettait d'enlever la couche de peau morte accumulée et d'améliorer ainsi le résultat de l'épilation.

Il est utile parfois d'effectuer un traitement de la peau également après l'arrachage, notamment par application d'un produit antiseptique, antirougeurs ou hydratant de la peau afin de fermer les pores et caimer la peau après l'épilation.

Ainsi, afin de parvenir à effectuer différents traitements des poils ou de la peau avant ou après l'épilation, avec un même appareil, une solution est de le munir d'accessoires interchangeables.

Le document WO 94/04116 décrit un appareil générateur de vapeur pour le traitement de la peau comportant un boîtier en forme de pistolet renfermant un moteur électrique dont l'arbre de sortie, agencé selon l'axe longitudinal du boîtier, fait saillie par rapport à ce dernier pour entraîner en rotation différents accessoires de traitement amovibles. L'un de ces accessoires est un dispositif d'épilation à ressorts, un autre est un disque abrasif entraîné en mouvement de rotation autour d'un axe perpendiculaire à la peau. Outre l'encombrement important de cet appareil et sa maniabilité peu aisée, on note que les dispositifs de traitement ont une action localisée sur la peau, plusieurs passages rapprochés étant nécessaires pour couvrir une zone de peau d'une largeur donnée, et, par conséquent, ils s'avèrent d'une efficacité limitée.

Une solution a été décrite dans le document EP 0 630 596 où l'appareil à épiler comporte un boîtier moteur et une roue de transmission apte à entraîner en rotation le cylindre rotatif à pinces d'arrachage de la tête à épiler. Selon ce document, la tête à épiler est montée de manière amovible par rapport au boîtier et elle peut être remplacée par une tête de tondeuse amovible. La tête de tondeuse amovible selon ce document renferme un mécanisme qui transforme le mouvement de rotation de la roue de transmission en un mouvement de va-et-vient du couteau mobile de la tondeuse et, de ce fait, elle s'avère d'un encombrement important.

Le document FR 2 728 777 décrit un dispositif de ponçage et polissage des callosités de la peau, dispositif qui est adaptable sur un boîtier moteur d'un appareil à épiler. Plus particulièrement, ce dispositif comporte une tête montée amovible par rapport au boîtier et à son entraînement, la tête comportant un cylindre abrasif muni d'un axe et d'un pignon d'entraînement venant directement en prise avec la roue de transmission du boîtier. La tête amovible ou, dans une variante, le boîtier de l'appareil fournissent les paliers de support de l'axe du cylindre abrasif. La tête de traitement selon ce document est certes de construction plus compacte que celle du document précédent, toutefois, le remplacement du cylindre abrasif n'est pas toujours facile à réaliser, car plusieurs opérations, voire même des outils, de démontage et de montage sont à prévoir. De surcroît, le cylindre étant réalisé en une pièce monobloc avec son axe, le coût de fabrication de tels accessoires interchangeables s'avère assez élevé.

On connaît par ailleurs, du document US 2002/0107527, un appareil de traitement de la peau par abrasion comportant un boîtier renfermant un moteur électrique et une transmission pour l'entraînement en un mouvement de rotation d'un cylindre abrasif autour d'un axe parallèle à la peau. Le rouleau abrasif est monté amovible par rapport au boîtier et à la transmission, pouvant être retiré de ce dernier ensemble avec sa tige d'entraînement. Pour remplacer le rouleau après l'avoir retiré du boîtier, il faut enlever d'abord la tige d'entraînement du rouleau, choisir un autre rouleau, le monter sur la tige, monter ensuite l'ensemble sur l'appareil, puis le fixer en utilisant un capuchon latéral de maintien de l'ensemble sur le boîtier. La solution constructive décrite dans ce document fait appel à plusieurs pièces démontables qui demandent plusieurs opérations successives de démontage puis de montage pour le remplacement du rouleau de traitement. Un tel appareil n'est nullement prévu pour recevoir une tête à épiler.

Le but de la présente invention est de remédier aux inconvénients précités et de fournir un appareil à épiler muni d'une tête à épiler et d'un ou plusieurs dispositifs de traitement de la peau qui puissent être inter changés de manière simple et aisée pour la personne l'utilisant.

Un autre but de l'invention est un dispositif de traitement de la peau dont les composants puissent être facilement nettoyés, échangés ou remplacés en effectuant un nombre restreint d'opérations de démontage et de montage par la personne utilisant l'appareil.

Un autre but de l'invention est un dispositif de traitement de la peau pour un appareil à épiler de conception optimisée et de durée de vie accrue dont les parties soumises à l'usure soient réalisées avec un nombre restreint de pièces, ceci dans un souci de protection de l'environnement et d'une facilité de remplacement de ces pièces.

Un autre but de l'invention est un dispositif de traitement de la peau qui soit de construction simple et fiable en fonctionnement et qui puisse être obtenu pour un coût de fabrication moindre.

Ces buts sont atteints avec un appareil à épiler comportant une tête à épiler, un dispositif de traitement de la peau et un boîtier formant moyen de préhension manuelle, renfermant un moteur électrique et un train d'engrenages recevant le mouvement du moteur pour actionner en rotation la tête à épiler montée de manière amovible par rapport au boîtier entre une position où elle reçoit le mouvement de rotation d'une roue de transmission du train d'engrenages et une autre position où elle est enlevée pour être remplacée par le dispositif de traitement de la peau monté également de manière amovible par rapport au boîtier et coopérant avec ladite roue de transmission, du fait que la tête à épiler comporte un cylindre rotatif à pinces d'arrachage entraîné en rotation autour d'un axe parallèle à la peau et que le dispositif de traitement de la peau comporte un rouleau de traitement monté directement amovible par rapport à une tige d'entraînement solidaire, elle, du dispositif de traitement.

Le dispositif de traitement de l'invention est monté amovible par rapport au boîtier d'un appareil à épiler, en lieu et place de la tête à épiler. Le dispositif de traitement coopère avec la roue dentée de transmission du train d'engrenages, c'est-à-dire que le dispositif comprend un pignon ou roue dentée qui s'engrène avec la roue de transmission pour transférer le mouvement de rotation au rouleau de traitement. Le dispositif de traitement comporte un rouleau de traitement monté rotatif autour d'un axe parallèle à la peau. Selon l'invention, le rouleau est monté directement amovible par rapport au boîtier ou au support du dispositif de traitement, plus particulièrement par rapport à une tige d'entraînement du rouleau, tige qui reçoit l'entraînement en rotation de la roue de transmission du train d'engrenages. On obtient ainsi un dispositif de traitement monté amovible par rapport au boîtier de l'appareil à épiler et comportant un rouleau monté également amovible par rapport à une tige d'entraînement en rotation du dispositif qui est, elle, montée fixe sur le boîtier ou support du dispositif de traitement. Ainsi, lorsque l'on veut retirer le dispositif de traitement du boîtier, on saisit le boîtier ou le support de celui-ci avec tous ses composants. Ensuite on peut encore désolidariser le rouleau de traitement par rapport au reste du dispositif. Ceci permet d'avoir un dispositif de traitement amovible dont le rouleau de traitement peut être facilement et directement retiré du dispositif, sans avoir à détacher d'autres pièces du dispositif, et facilement remplacé par un autre rouleau en l'enclenchant directement sur la tige d'entraînement du dispositif.

Par rouleau monté directement amovible par rapport à une tige d'entraînement en rotation, on comprend qu'au moins l'une des opérations de montage et/ou de démontage du rouleau se fait à la main, sans utiliser d'outils de montage ou de démontage du rouleau par rapport à son support d'entraînement.

Ainsi, le rouleau peut rapidement être attaché à son support et respectivement être facilement retiré de son support en vue de son remplacement, nettoyage, etc., tout en étant relié de manière sûre à son support lors de son entraînement en rotation. Le rouleau peut donc tourner à grande vitesse (-2000 rot/min) avec son support, en étant entraîné par ce dernier, sans qu'il y ait de mouvement relatif entre les deux.

De préférence, ladite tige d'entraînement est solidaire d'une roue dentée ou pignon du dispositif de traitement coopérant avec la roue de transmission.

Un entraînement par une roue dentée permet déjà de transmettre un couple de rotation important nécessaire pour l'entraînement d'une tête à épiler comportant des pinces d'arrachage. Il est avantageux par la suite d'utiliser la même roue dentée pour entraîner d'autres dispositifs de traitement amovibles venant en remplacement de la tête à épiler amovible.

De préférence, ladite tige d'entraînement est supportée en rotation par les branches d'un cadre en U du dispositif de traitement.

Un tel cadre forme support pour la tige d'entraînement et son rouleau de traitement, le cadre étant fixé au boîtier par sa base et les branches latérales permettant à la tige d'être supportée en rotation. Par ailleurs, les branches du cadre peuvent être facilement saisies à la main par l'utilisateur pour monter le cadre au boîtier, la base du cadre ayant avantageusement les mêmes dimensions que l'ouverture de la partie supérieure du boîtier.

Avantageusement, ladite tige d'entraînement est réalisée en une pièce avec deux flasques latéraux et forme une bobine d'entraînement, l'un desdits flasques comportant un pignon d'entraînement pour la mise en rotation de la bobine d'entraînement depuis la roue de transmission.

Une telle bobine d'entraînement assure l'entraînement direct du rouleau depuis la roue de transmission du boîtier, pour une solution constructive simplifiée. Cette bobine est supportée en rotation par le cadre en U du dispositif de traitement.

Utilement, ladite bobine d'entraînement tourne autour d'une broche montée fixe dans les évidements des deux parois latérales amovibles du dispositif de traitement.

Avantageusement, ledit rouleau ou ladite tige d'entraînement comprennent des moyens d'accouplement élastiques.

Par moyens d'accouplement élastiques, on comprend des moyens permettant un accouplement par déformation d'une première pièce, d'entraînement ou entraînée, autour d'une deuxième pièce, qui est alors entraînée, respectivement d'entraînement, sous l'effet d'une sollicitation mécanique, première pièce qui reprend, de par ses propriétés d'élasticité, sa forme initiale dès que l'effort qui lui était appliqué a cessé. Ce type d'accouplement permet une fixation manuelle rapide des deux pièces.

De préférence, ledit rouleau est fendu longitudinalement, les bords d'une fente longitudinale étant tenus ensemble par une pince radiale.

On aurait pu, certes, envisager une tige d'entraînement avec une fente sur laquelle s'engagerait une nervure d'entraînement du rouleau de traitement. Pour des raisons de facilité de montage et de démontage du rouleau, on préfère toutefois réaliser un rouleau de traitement fendu et évidé, les bords de la fente étant alors tenus ensemble par une pince radiale située à l'intérieur du rouleau de traitement. Ceci permet un montage et un démontage faciles et rapides depuis l'extérieur du rouleau directement sur la tige d'entraînement, sans outils et sans avoir à monter ou à démonter par la suite d'autres composants, tels des butées axiales, etc.

Utilement, ladite tige d'entraînement comporte au moins une ailette d'entraînement venant en prise avec les bords de ladite pince radiale, ceci afin d'assurer un entraînement efficace, sans mouvement relatif entre la tige d'entraînement et le rouleau entraîné.

Avantageusement, ladite pince radiale comporte un clip de fixation sur ladite tige d'entraînement.

Une telle pince radiale agencée à l'intérieur du corps du rouleau évidé présente en sa partie centrale un clip de fixation qui, de par sa forme et ses propriétés d'élasticité, s'engage autour de la tige d'entraînement assurant ainsi la fixation du rouleau autour de la tige.

De préférence, ladite pince radiale comporte deux branches s'étendant de part et d'autre du clip pour se rejoindre en la partie diamétralement opposée à celle de ladite fente.

On aurait pu envisager une pince radiale à deux branches qui relient les bords de la fente et se rejoignent en partie centrale, autour de l'axe du rouleau, où elles forment le clip de fixation sur la tige d'entraînement. On préfère toutefois une pince radiale agencée à l'intérieur du rouleau de traitement, en étant reliée aux bords de la fente et se prolongeant jusqu'en partie diamétralement opposée, car cette solution confère plus d'élasticité au rouleau, l'angle d'ouverture étant plus faible, la matière du corps du rouleau est moins sollicitée. Cette solution permet également à la pince ainsi réalisée de venir en prise avec deux nervures d'entraînement diamétralement opposées de la tige d'entraînement.

Avantageusement, ledit rouleau comprend un corps recouvert extérieurement d'un revêtement.

Ceci permet d'obtenir plusieurs types de rouleaux de traitement en utilisant un même moule pour le corps sur lequel on vient appliquer divers revêtements par la suite.

Dans une variante de l'invention, ledit revêtement comprend un matériau abrasif.

Ainsi, un rouleau de traitement comportant un revêtement abrasif peut être utilisé pour réaliser un gommage de la peau avant épilation. Le type de matériau abrasif est choisi en fonction de la typologie de la peau de la personne pour réaliser un gommage toléré par la peau. Un tel dispositif de traitement pouvant par ailleurs être utilisé pour éliminer les callosités ou pour limer les ongles, la granulométrie du matériau abrasif sera donc choisie également en fonction du type de traitement à effectuer avec ce dispositif.

Dans d'autres variantes de l'invention, le rouleau de traitement peut comporter d'autres types de revêtements, notamment un revêtement textile (déposé par collage d'un tissu, par flocage de fibres textiles sur la surface du rouleau, etc.), ou un revêtement céramique ou métallique, voire un revêtement à protubérances de massage, etc.

Un tel dispositif de traitement est réalisé à base de pièces amovibles, ce qui permet, à la fois, un assemblage aisé et un démontage facilité pour le remplacement des pièces usées ou même pour le recyclage des pièces plastiques et des pièces métalliques séparément.

L'invention sera mieux comprise à l'étude des modes de réalisation pris à titre nullement limitatif et illustrés dans les figures annexées dans lesquelles :
- la figure 1 est une vue en perspective d'un appareil à épiler muni d'une tête à épiler selon un exemple de réalisation de l'invention, une partie des éléments constituant le boîtier de la tête à épiler étant enlevés;
- la figure 2 est une vue en perspective d'un dispositif de traitement selon un exemple de réalisation de l'invention;
- la figure 3 est une vue en perspective d'un outil de démontage utilisé avec le dispositif de traitement de la figure 2;
- la figure 4 est une vue éclatée du dispositif de traitement de la figure 2;
- la figure 5 est une vue en coupe axiale du dispositif de traitement de la figure 2 réalisée avec un plan A-A vertical passant par l'axe longitudinal du dispositif.

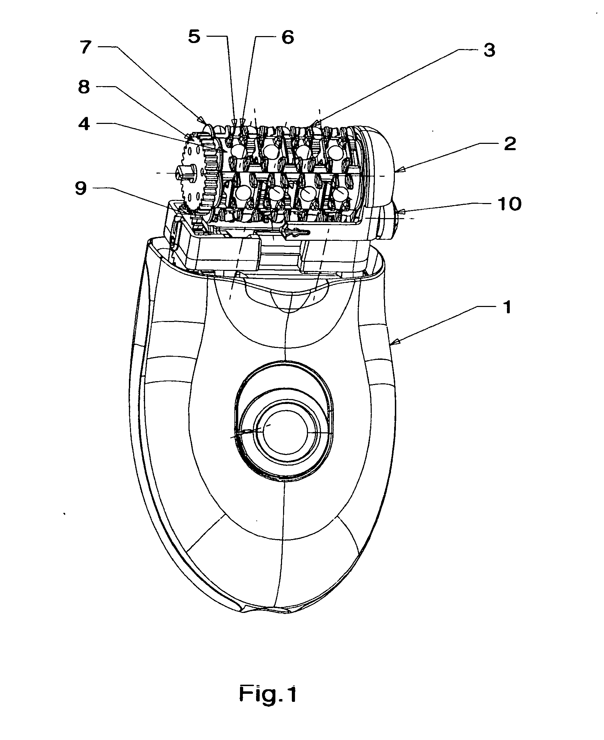

L'appareil à épiler représenté à la figure 1 comprend un boîtier 1 et une tête à épiler 2. Le boîtier 1 renferme un moteur électrique et un train d'engrenages qui transmet le mouvement de rotation de l'arbre de sortie du moteur à une roue de transmission 9 venant directement en prise avec un pignon d'entraînement 8 du cylindre rotatif 3 de la tête à épiler 2. Le cache gauche du boîtier de la tête à épiler 2 a été enlevé dans la représentation de la figure 1 afin de laisser apparaître une partie des éléments constitutifs de l'appareil ici décrit. Le cylindre rotatif 3 comprend plusieurs modules d'arrachage 4 portés par une cage périphérique 7 concentrique à l'axe longitudinal du cylindre rotatif 3 et entraînée en rotation par le pignon d'entraînement 8 de ce dernier. Un module d'arrachage 4 comprend plusieurs pinces d'arrachage, une pince étant formée de lames mobiles 5 et de lames fixes 6. La base d'une lame mobile 5 suit la rainure d'une came interne et s'applique et s'éloigne successivement d'une lame fixe 6 adjacente lors de son entraînement en rotation autour de l'axe longitudinal du cylindre rotatif 3. Les pinces des modules d'arrachage 4 sont décalées angulairement sur la périphérie du cylindre rotatif 3. En fonctionnement, lorsque le moteur électrique est alimenté, il met en rotation le cylindre rotatif 3 dont les pinces se ferment et s'ouvrent successivement et arrachent les poils de la zone à épiler qui se présentent devant la tête à épiler. Un tel cylindre rotatif est mieux décrit dans la demande de brevet FR 2 858 528 au nom de la demanderesse et constitue un exemple de réalisation d'appareil à épiler selon l'invention.

La tête à épiler 2 est montée de manière amovible par rapport au boîtier 1 et à la roue de transmission 9 de ce dernier. Pour ceci, la tête à épiler 2 et le boîtier 1 sont munis de moyens de verrouillage à l'aide desquels ils sont fixés l'un à l'autre et sont ensuite désolidarisés l'un par rapport à l'autre. Ainsi, la tête à épiler peut être munie de tenons venant automatiquement en prise avec des languettes de verrouillage du boîtier. Un bouton de déverrouillage 10 est prévu sur un côté du boîtier 1 à l'aide duquel les tenons de la tête à épiler sont libérés et la tête à épiler peut être retirée du boîtier 1.

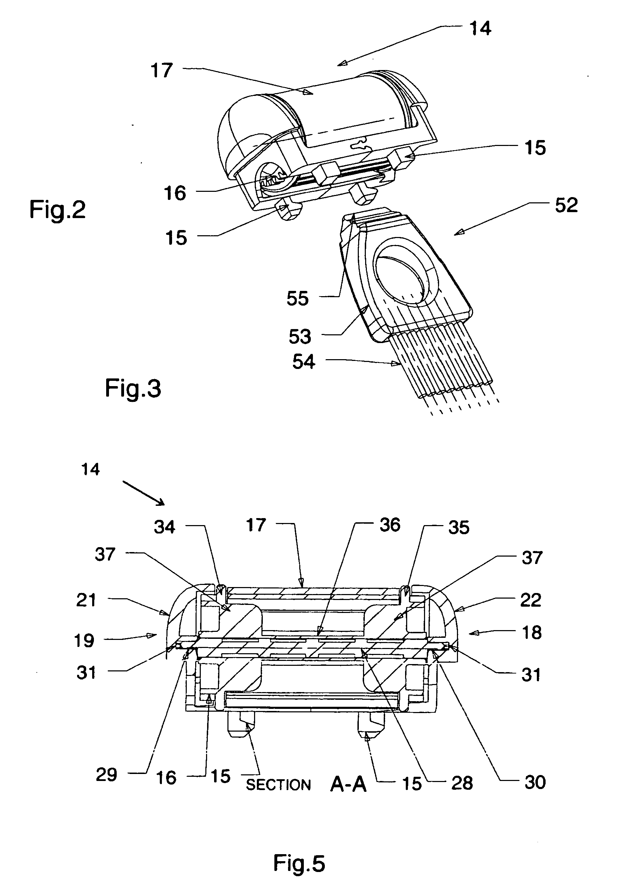

La figure 2 représente un dispositif de traitement 14 de la peau qui est apte à être monté en lieu et place de la tête à épiler 2. Le dispositif de traitement 14 présente, en sa partie inférieure, des tenons 15 venant en prise avec des languettes prévues à cet effet dans le boîtier 1 de l'appareil, le bouton de déverrouillage 10 permettant aux tenons 15 d'être libérés et au dispositif de traitement 14 d'être retiré du boîtier 1. Un pignon d'entraînement 16 en rotation d'un rouleau 17 est prévu sur au moins l'un des côtés latéraux du dispositif de traitement 14, ce pignon étant apte à venir en prise avec la roue de transmission 9 du boîtier 1.

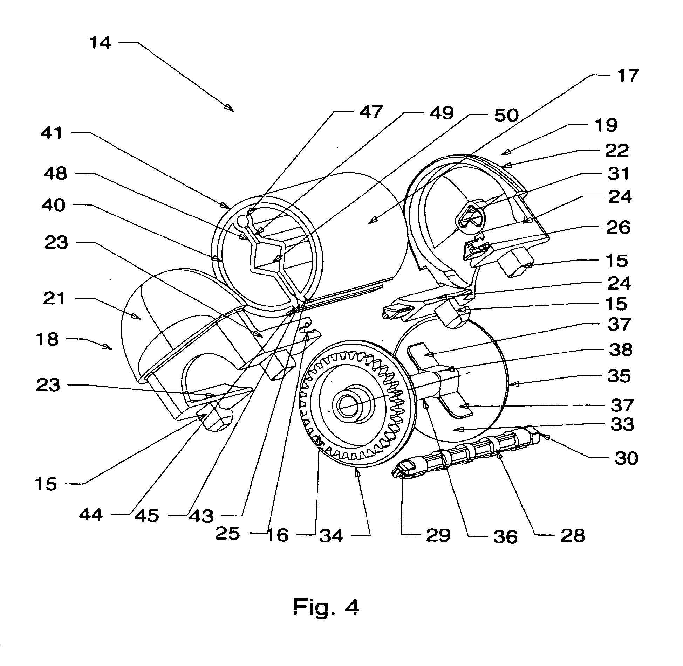

Tel que mieux visible aux figures 4 et 5, le dispositif de traitement 14 comprend deux caches latéraux, notamment un cache latéral gauche 18 et un cache latéral droit 19, ayant chacun un profil en L. Chaque paroi latérale, notamment la paroi latérale 21 du cache gauche 18 et la paroi latérale 22 du cache droit 19, se prolonge par deux bras transversaux parallèles, notamment deux bras 23 à gauche et respectivement deux bras 24 à droite. Les bras 23 et les bras 24 se trouvent les uns dans le prolongement des autres et comprennent des moyens d'assemblage à leurs extrémités respectives. Plus particulièrement, les bras 23 comportent des rainures 25 servant de fixation aux nervures 26 situées à l'extrémité des bras 24. Les parois latérales 21,22 et leurs bras 23,24 forment un cadre ayant une forme générale en U. Les parois latérales 21 et 22 supportent en la partie centrale de leur face interne une broche 28 métallique, de forme globalement cylindrique ou présentant des paliers de forme cylindrique sur sa longueur. Les extrémités 29,30 de la broche 28 ont une forme en coin et sont montées fixes, sans possibilité de rotation, dans deux évidements 31 de forme correspondante pratiqués dans les parois latérales 21,22.

La broche 28 supporte en rotation une bobine d'entraînement 33 comportant deux flasques 34, 35 en forme de disque reliés en leur centre par une tige 36 tubulaire. La bobine d'entraînement 33 tourne autour de la broche 28 en étant entraînée en rotation par le pignon 16 solidaire de la face externe du flasque 34 venant en prise avec la roue de transmission 9 du boîtier 1. Les parties de jonction de la tige 36 avec les faces internes des flasques 34 et 35 sont réalisées chacune sous forme d'un moyeu 38 en forme de prisme de section carré se prolongeant vers la périphérie des flasques par deux ailettes 37 diamétralement opposées. Le contour carré du moyeu 38 est circonscrit à celui circulaire de la tige 36, les ailettes 37, disposées selon l'une des diagonales de la section carrée du moyeu 38, sont perpendiculaires au plan des faces internes des flasques 34,35, les sommets situés sur l'autre diagonale comportant, eux, des rayons de courbure afin de faciliter l'introduction d'un rouleau 17, tel qu'il sera expliqué par la suite. La bobine d'entraînement 33 est réalisée de préférence en un matériau plastique par une technique d'injection.

Un rouleau 17 de traitement comprend un corps 40 cylindrique et un revêtement 41 le recouvrant sur sa surface latérale. Le corps 40 a une forme de cylindre évidé et fendu le long de sa génératrice. Les bords 43,44 délimitant une fente 45 sur la surface latérale du corps 40 sont tenus ensemble par une pince radiale 47 qui s'étend sur toute la longueur du corps 40. La section transversale du corps 40 présente un contour fermé lui assurant à la fois de bonnes propriétés d'élasticité et suffisamment de rigidité pour une bonne tenue aux déformations. Les bords 43,44 sont saillants par rapport à la surface latérale du corps 40.

En section transversale, la pince radiale 47 est une tige recourbée à deux branches 48,49 parallèles et symétriques par rapport à un plan médian passant par l'axe du corps 40. Chaque branche 48,49 est solidaire à l'une de ses extrémités de l'un des bords 43,44 de la fente 45 et se prolonge en direction radiale pour former en partie centrale un V couché d'angle droit au sommet. Les deux branches se prolongent toujours en direction radiale et se rejoignent en arc de cercle sur la face interne du corps 40, situé diamétralement opposé par rapport aux bords 43, 44 de la fente 45. Les deux parties en V se faisant face forment, en partie centrale et de préférence sur toute la longueur du rouleau 17, un clip 50 élastique de fixation de ce dernier sur la tige 36 de la bobine d'entraînement 33. Deux fentes latérales sont ainsi formées, de part et d'autre du clip 50 et dans le prolongement de la fente 45 et permettent d'accueillir les languettes 37 en vue de l'entraînement en rotation du rouleau 17. La longueur des branches 48, 49 de la pince 47 détermine l'angle d'ouverture de la fente 45, et donc pour une longueur plus importante des branches 48, 49, l'angle d'ouverture étant plus faible, la matière du corps 40 du rouleau est moins sollicitée et la durée de vie se trouve ainsi améliorée.

Dans une variante de l'invention, les branches 48, 49 de la pince radiale 47 relient les bords 43, 44 de la fente 45 et se rejoignent en partie centrale du corps 40, après avoir formé ensemble le clip 50. Dans ce cas, une seule fente latérale est formée, sur un côté du clip 50, cette fente latérale venant en prise avec une languette 37 située, elle aussi, sur un côté de la tige d'entraînement 36.

Le corps 40 du rouleau 17 présentant une section transversale de forme complexe et d'épaisseur constante, est avantageusement réalisé en une matière plastique, tel l'ABS ou le POM, par une technique d'injection ou d'extrusion. Le corps 40 pourrait également être réalisé en métal, par exemple inox, aluminium, etc. à partir d'un profilé extrudé. A titre d'exemple, l'épaisseur du corps 40 du rouleau et de la pince 47 qu'il renferme, peut être avantageusement comprise entre 0.5mm et 1 mm.

Dans une variante de l'invention, le revêtement 41 est une feuille de matériau abrasif appliquée par collage sur la surface latérale du corps 40. La feuille de matériau abrasif appliquée doit avoir une épaisseur au moins égale à celle de la partie saillante des bords 43,44 de manière à former avec ces derniers une surface continue afin de ne pas heurter la peau. Afin d'assurer un contact encore plus doux avec la peau, la feuille de matériau adhésif peut être appliquée moyennant une couche de mousse sur le corps 40. La granulométrie du matériau abrasif ou le type de revêtement 41 sont choisis en fonction de la typologie de la peau ou du type de traitement à effectuer.

L'assemblage du dispositif de traitement 14 se fait en fixant d'abord l'extrémité 29 de la broche 28 dans l'orifice 31 de la paroi latérale 21. On fait ensuite glisser la bobine d'entraînement 33 sur la broche 28 et on ramène à l'ensemble l'autre paroi latérale 22, en fixant l'extrémité 30 de la broche 28 dans l'orifice 31 de la paroi 22. Le sous-ensemble ainsi obtenu peut déjà être monté au boîtier de l'appareil, le rouleau 17 pouvant être attaché par la suite sur la tige 36. Pour ceci, on oriente les ailettes 37 de la bobine d'entraînement vers la fente 45 du rouleau 17 et on enclenche de force le rouleau jusqu'à ce que les ailettes 37 soient en place et le clip 50 accroche la tige 36 de la bobine d'entraînement 33. Lorsque le moteur électrique de l'appareil est alimenté, le mouvement de rotation reçu de la roue de transmission 9 met en rotation la bobine d'entraînement 33 autour de la broche 28, qui, elle, est montée fixe parallèlement à la peau, et les ailettes 37 de la bobine d'entraînement 33 font alors tourner le rouleau 17.

Pour retirer le rouleau 17 du dispositif de traitement 14, il suffit d'écarter les bords 43, 44 de la fente 45 à la main ou à l'aide d'un outil. La figure 3 montre une brosse 52 comportant un corps 53 muni, à l'une de ses extrémités, d'une ou plusieurs rangées de poils 54. Le corps 53 est muni à l'extrémité opposée à celle portant les poils 54, d'un rebord 55 formant une languette d'épaisseur légèrement inférieure à celle de la fente 45 du rouleau 17. Ainsi, pour faciliter davantage le démontage du rouleau 17, on insère le rebord 55 de la brosse 52 dans la fente 45 du rouleau 17, on écarte les bords 43, 44 de cette dernière en tirant en même temps le rouleau 17 vers l'extérieur pour le désolidariser de la bobine d'entraînement 33. La brosse 52 est par ailleurs utilisée pour nettoyer la tête d'épilation 2 ou le dispositif de traitement 14.

Le dispositif de traitement de l'invention permet un remplacement facile et rapide des rouleaux 17 qui peuvent ainsi être retirés et remplacés par d'autres remplissant des fonctions différentes ou adaptées à des buts spécifiques. Ainsi, plusieurs rouleaux 17 ayant chacun une granulométrie spécifique ou un revêtement spécifique sont aptes à être utilisés avec le dispositif de traitement de l'invention en étant facilement interchangeables.

Dans une variante de l'invention, le dispositif de traitement 14 comporte un rouleau 17 dont le revêtement 41 est un tissu ou une mousse imbibés d'un liquide du type produit antiseptique, antirougeurs, hydratant ou calmant de la peau. De surcroît, un réservoir de produit à appliquer sur la peau et des moyens de mise en communication avec le rouleau 17 peuvent être prévus à l'intérieur du boîtier de l'appareil ou du dispositif de traitement.

Dans une autre variante de l'invention, le rouleau 17 renferme un réservoir de matériau à changement de phase qui, lorsque mis au congélateur, accumule le froid. Le rouleau comporte alors un revêtement lisse qui est en communication thermique avec le réservoir et produit un effet calmant ou antidouleur lorsqu'il est appliqué sur la peau.

Dans une autre variante, ledit revêtement peut être du type non adhésif ou céramique ou à changement de couleur lorsque chauffé. Des moyens de chauffe du rouleau, électriques ou à réaction exothermique, peuvent également être prévus.

Dans une autre variante le rouleau de traitement est destiné au massage de la peau et il comporte dans ce but des protubérances sur sa surface.

Dans une autre variante, le rouleau de traitement est destiné à limer les ongles ou les callosités de la peau. Le rouleau est alors recouvert d'un matériau abrasif adapté à ce type de traitement.

D'autres variantes et modes de réalisation de l'invention peuvent être envisagés sans sortir du cadre de ces revendications.

Ainsi, l'appareil à épiler peut comprendre deux cylindres rotatifs côte-à-côte, par exemple un cylindre d'épilation et un rouleau de traitement ou deux rouleaux de traitement côte-à-côte, entraînés chacun par la ou par une roue de transmission sortant du boîtier.

## Revendications

1. Appareil à épiler comportant une tête à épiler (2), un dispositif de traitement (14) de la peau et un boîtier (1) formant moyen de préhension manuelle, renfermant un moteur électrique et un train d'engrenages recevant le mouvement du moteur pour actionner en rotation la tête à épiler (2) montée de manière amovible par rapport au boîtier entre une position où elle reçoit le mouvement de rotation d'une roue de transmission (9) du train d'engrenages et une autre position où elle est enlevée pour être remplacée par le dispositif de traitement (14) de la peau monté également de manière amovible par rapport au boîtier et coopérant avec ladite roue de transmission (9), **caractérisé en ce que** la tête à épiler (2) comporte un cylindre rotatif à pinces d'arrachage entraîné en rotation autour d'un axe parallèle à la peau et que le dispositif de traitement de la peau comporte un rouleau (17) de traitement monté directement amovible par rapport à une tige (36) d'entraînement solidaire, elle, du dispositif de traitement.

2. Appareil selon la revendication 1, **caractérisé en ce que** ladite tige (36) d'entraînement est solidaire d'une roue dentée ou pignon du dispositif de traitement (14) coopérant avec la roue de transmission (9).

3. Appareil selon l'une des revendications 1 ou 2, **caractérisé en ce que** ladite tige (36) d'entraînement est supportée en rotation par les branches d'un cadre en U du dispositif de traitement (14).

4. Appareil selon l'une des revendications précédentes, **caractérisé en ce que** ladite tige d'entraînement (36) est réalisée en une pièce avec deux flasques (34,35) latéraux et forme une bobine d'entraînement (33), l'un desdits flasques comportant un pignon d'entraînement (16) pour la mise en rotation de la bobine d'entraînement (33) depuis la roue de transmission (9).

5. Appareil selon la revendication 4, **caractérisé en ce que** ladite bobine d'entraînement (33) tourne autour d'une broche (28) montée fixe dans les évidements (31) des deux parois latérales (21,22) amovibles du dispositif de traitement (14).

6. Appareil selon l'une des revendications précédentes, **caractérisé en ce que** ledit rouleau (17) ou ladite tige (36) d'entraînement comprennent des moyens d'accouplement élastiques.

7. Appareil selon l'une des revendications précédentes, **caractérisé en ce que** ledit rouleau (17) est fendu longitudinalement, les bords (43,44) d'une fente (45) longitudinale étant tenus ensemble par une pince radiale (47).

8. Appareil selon la revendication 7, **caractérisé en ce que** ladite tige (36) d'entraînement comporte au moins une ailette (37) d'entraînement venant en prise avec les bords de ladite pince radiale (47).

9. Appareil selon l'une des revendications 7 ou 8, **caractérisé en ce que** ladite pince radiale (47) comporte un clip (50) de fixation sur ladite tige (36) d'entraînement.

10. Appareil selon l'une des revendications 7 à 9, **caractérisé en ce que** ladite pince radiale (47) comporte deux branches (48,49) s'étendant de part et d'autre du clip (50) pour se rejoindre en la partie diamétralement opposée à celle de ladite fente (45).

11. Appareil selon l'une des revendications précédentes, **caractérisé en ce que** ledit rouleau (17) comprend un corps (40) recouvert extérieurement d'un revêtement (41).

12. Appareil selon la revendication 11, **caractérisé en ce que** ledit revêtement (41) comprend un matériau abrasif.

## Claims

1. An epilator appliance comprising an epilator head (2), a skin treatment device (14), and a housing (1) forming hand-grip means and containing an electric motor and a gear train receiving drive from the motor for rotary actuation of the epilator head (2) that is mounted removably relative to the housing between a position in which it receives rotary drive from a transmission wheel (9) of the gear train, and another position in which it is removed in order to be replaced by the skin treatment device (14), also mounted removably relative to the housing and co-operating with said transmission wheel (9), the appliance being **characterized in that** the epilator head (2) comprises a rotary cylinder of plucking pincers driven in rotation about an axis parallel to the skin, and that the skin treatment device comprises a treatment roller (17) mounted to be directly removable relative to a drive rod (36) that is itself secured to the treatment device.

2. An appliance according to claim 1, **characterized in that** said drive rod (36) is secured to a toothed wheel or gearwheel of the treatment device (14) co-operating with the transmission wheel (9).

3. An appliance according to claim 1 or claim 2, **characterized in that** said drive rod (36) is supported in rotation by the branches of a U-shaped frame of the treatment device (14).

4. An appliance according to any preceding claim, **characterized in that** said drive rod (36) is made integrally with two side plates (34, 35) and forms a drive spool (33), one of said side plates including a drive gearwheel (16) for setting the drive spool (33) into rotation from the transmission wheel (9).

5. An appliance according to claim 4, **characterized in that** said drive spool (33) turns about a spindle (28) mounted permanently in the recesses (31) of the two removable side walls (21, 22) of the treatment device (14).

6. An appliance according to any preceding claim, **characterized in that** said roller (17) or said drive rod (36) includes resilient coupling means.

7. An appliance according to any preceding claim, **characterized in that** said roller (17) is slotted longitudinally, the edges (43, 44) of a longitudinal slot (45) being held together by a radial clamp (47).

8. An appliance according to claim 7, **characterized in that** said drive rod (36) includes at least one drive fin (37) engaging with the edges of said radial clamp (47).

9. An appliance according to claim 7 or claim 8, **characterized in that** said radial clamp (47) comprises a clip (50) for fastening on said drive rod (36).

10. An appliance according to any one of claims 7 to 9, **characterized in that** said radial clamp (47) comprises two branches (48, 49) extending on either side of the clip (50) and meeting in the portion that is diametrically opposite from the portion containing said slot (45).

11. An appliance according to any preceding claim, **characterized in that** said roller (17) comprises a body (40) externally covered in a covering (41).

12. An appliance according to claim 11, **characterized in that** said covering (41) comprises an abrasive material.

## Patentansprüche

1. Epiliergerät mit einem Epilierkopf (2), einer Vorrichtung zum Behandeln (14) der Haut und einem Gehäuse (1), das ein Mittel zum Greifen mit der Hand bildet und einen Elektromotor und einen Getriebezug einschließt, der die Bewegung des Motors aufnimmt, um den Epilierkopf (2) in Drehung zu versetzen, der bezüglich des Gehäuses zwischen einer Stellung abnehmbar angebracht ist, in welcher er die Drehbewegung eines Getrieberads (9) des Getriebezugs (9) aufnimmt, und einer weiteren Stellung, in welcher er entfernt ist, um durch die Vorrichtung zum Behandeln der Haut (14) ersetzt zu werden, die ebenfalls bezüglich des Gehäuses abnehmbar angebracht ist und mit dem Getrieberad (9) zusammenwirkt, **dadurch gekennzeichnet, dass** der Epilierkopf (2) einen drehenden Zylinder mit Ausreißzangen aufweist, die um eine zur Haut parallele Achse in Drehung versetzt ist, und dass die Vorrichtung zum Behandeln der Haut eine Behandlungswalze (17) aufweist, die bezüglich einer Antriebsstange (36) unmittelbar abnehmbar angebracht ist, die wiederum mit der Behandlungsvorrichtung fest verbunden ist.

2. Gerät nach Anspruch 1, **dadurch gekennzeichnet, dass** die Antriebsstange (36) mit einem Zahnrad oder Ritzel der Behandlungsvorrichtung (14) fest verbunden ist, welches mit dem Getrieberad (9) zusammenwirkt.

3. Gerät nach Anspruch 1 oder 2, **dadurch gekennzeichnet, dass** die Antriebsstange (36) durch die Schenkel eines U-förmigen Rahmens der Behandlungsvorrichtung (14) in Drehung gestützt ist.

4. Gerät nach einem der vorhergehenden Ansprüche, **dadurch gekennzeichnet, dass** die Antriebsstange (36) aus einem Stück mit zwei seitlichen Flanschen (34, 35) ausgebildet ist und eine Antriebsspule (33) bildet, wobei einer der Flansche ein Antriebsritzel (16) aufweist, um die Antriebsspule (33) ausgehend von dem Getrieberad (9) in Drehung zu versetzen.

5. Gerät nach Anspruch 4, **dadurch gekennzeichnet, dass** die Antriebsspule (33) um einen Spindel (28) dreht, der in den Aussparungen (31) der beiden abnehmbaren Seitenwände (21, 22) der Behandlungsvorrichtung (14) fest angebracht ist.

6. Gerät nach einem der vorhergehenden Ansprüche, **dadurch gekennzeichnet, dass** die Walze (17) oder die Antriebsstange (36) elastische Kupplungsmittel aufweisen.

7. Gerät nach einem der vorhergehenden Ansprüche, **dadurch gekennzeichnet, dass** die Walze (17) längs geschlitzt ist, wobei die Ränder (43, 44) eines Längsschlitzes (45) durch eine radiale Klammer (47) zusammengehalten sind.

8. Gerät nach Anspruch 7, **dadurch gekennzeichnet, dass** die Antriebsstange (36) mindestens einen Antriebsflügel (37) aufweist, der mit den Rändern der radialen Klammer (47) in Eingriff kommen.

9. Gerät nach Anspruch 7 oder 8, **dadurch gekennzeichnet, dass** die radiale Klammer (47) einen Clip (50) zur Befestigung auf der Antriebsstange (36) aufweist.

10. Gerät nach einem der Ansprüche 7 oder 9, **dadurch gekennzeichnet, dass** die radiale Klammer (47) zwei Schenkel (48, 49) aufweist, die sich auf der einen und anderen Seite des Clips (50) erstrecken, um in dem Teil zusammenzulaufen, der dem Schlitz (45) diametral gegenüberliegt.

11. Gerät nach einem der vorhergehenden Ansprüche, **dadurch gekennzeichnet, dass** die Walze (17) einen Körper (40) aufweist, der außen mit einer Beschichtung (41) überzogen ist.

12. Gerät nach Anspruch 11, **dadurch gekennzeichnet, dass** die Beschichtung (41) ein Schleifmittel aufweist.
